# EUROPÄISCHE PATENTSCHRIFT

(11) **EP 2 060 297 B1**
(45) Veröffentlichungstag und Bekanntmachung des Hinweises auf die Patenterteilung: **08.10.2014**
(21) Anmeldenummer: 08169115.6
(22) Anmeldetag: 14.11.2008
(51) Int. Cl.: A61M 25/02, A61M 16/04

(54) **Fixierband für Endotrachealtubus**
Fixing ribbon for endo-tracheal tube
Bande de fixation pour un tube endotrachéal

(30) Priorität: 16.11.2007 DE 202007016046 U; 08.02.2008 DE 202008001769 U
(43) Veröffentlichungstag der Anmeldung: 20.05.2009
(73) Patentinhaber: Technicare Establishment, 9490 Vaduz (LI)
(72) Erfinder: Piwonka, Peter, 9400 Rohrschach (CH)
(74) Vertreter: Körber, Martin Hans

(56) Entgegenhaltungen:
- DE-U1- 8 712 347
- DE-U1-202005 015 555
- DE-U1-202007 008 218

## Beschreibung

Die Erfindung betrifft ein Fixierband gemäß dem Oberbegriff des Hauptanspruches. Derartige Fixierbänder werden verwendet, um bei beatmeten Patienten Endotrachealtuben gegen eine ungewollte Lageveränderung zu sichern. Wenn ein gelegter Endotrachealtubus verrutscht, kann es schlimmstenfalls zu lebensbedrohlichen Situationen für den beatmeten Patienten kommen.

Bisher ist bekannt, wie in dem unter DE 8712347 U1 veröffentlichten Gebrauchsmuster beschrieben, ein Fixierband zur Positionierung um einen Endotrachealtubus zu schlingen und das Band im Weiteren um den Kopf des Patienten zu legen. Dieses Band wird aus Schaumstoff gefertigt.

Nachteilig hierbei ist, dass das Fixierband nur durch die Reibwirkung des Fixierbandes selbst am Endotrachealtubus fixiert ist, wobei Schaumstoff keinen besonderen Reibschluss erzeugt, wie dies beispielsweise unter Einsatz von Klebstoff der Fall wäre. Damit besteht die Gefahr, dass der Endotrachealtubus im Fixierband wandern kann, was vor allem durch Husten oder dem Atemdruck am Endotrachealtubus ausgelöst wird.

Ein weiterer Nachteil besteht darin, dass sich das aus Schaumstoff gefertigte Band, durch das unmittelbare anliegen an Gesicht und Hals des Patienten, mit Flüssigkeit vor allem Speichel, Sekret und Schweiß, voll saugen kann.

Damit entwickelt das Fixierband eine Art Schwammwirkung, was in der Folge die Gesichtshaut bei längerem Belassen eines feuchten Fixierbandes beeinträchtigt. Ein dadurch notwendiger häufiger Wechsel mit einem neuen trockenen Fixierband verursacht aber erhöhten Arbeitsaufwand und Kosten für den Anwender. Auch zeigt in der Praxis ein aus Schaumstoff gefertigtes schwammartig vollgesaugtes Fixierband aus Schaumstoffmaterial einen Dehnungseffekt. Damit ist ein solch vollgesaugtes Fixierband ein Sicherheitsrisiko für den beatmeten Patienten, da unter diesen Umständen die Lagefixierung des Endotrachealtubus zumindest gelockert ist. Der Endotrachealtubus kann sich dann von selbst aus der angelegten Position ändern. Ein zusätzliches Problem bei allen ansonsten eingesetzten Bändern ist auch, dass sich der Endotrachealtubus nicht auf einer bestimmten Seite des Mundes befestigen lässt, das heißt der Tubus kann unter Unständen unkontrolliert in den Mundwinkeln von rechts nach links wandern, was in ungünstigen Fällen zu Druckgeschwüren führen kann.

Weiter ist es gebräuchlich, den Tubus mittels Pflaster, z. B. Pflasterstreifen, zu fixieren. Das generelle Problem dabei ist, dass Pflaster Allergien hervorrufen können und bei Patienten mit Schweiß, Bart oder Verletzungen im Gesichtsbereich nicht ausreichend haltbar angebracht werden können.

So besteht die Aufgabe der vorliegenden Erfindung darin, eine Fixierung für Endotrachealtuben zu schaffen, welche einen möglichst sicheren Sitz des eingeführten Endotrachealtubus gewährleistet, ohne dabei auf die Haut des betroffenen Patienten einzuwirken und gleichzeitig den Verbrauch an Fixierbändern zu senken.

Die Aufgabe wird erfindungsgemäß gelöst durch das kennzeichnende Merkmal des Hauptanspruchs. Vorteilhafte Ausführungsformen ergeben sich aus den Unteransprüchen.

Das Fixierband hat eine Oberseite und eine Unterseite, wobei nur die Unterseite eine hydrophobe Beschichtung aufweist. Diese Beschichtung kann vollflächig oder aber in Streifen erfolgen. Damit kann das Fixierband, gleich aus welchem Material es gefertigt ist, keine Flüssigkeit abgeben, sofern die beschichtete Seite unmittelbar an der Haut des Patienten geführt wird. Ungekehrt wird ein Vollsaugen erschwert. Ein weiterer Vorteil einer Beschichtung ist, dass das Fixierband dadurch einen anderen höheren Reibkoeffizienten hat und damit rutschfester am Endotrachealtubus angebracht werden kann.

Vorteilhafterweise weist das Fixierband nur auf einer der beiden Seiten, beispielsweise der Unterseite, die hydrophobe Beschichtung auf. Hierbei kann die Oberseite des Fixierbandes vorteilhafterweise als ein Teil eines Klettverschlusses ausgebildet sein, wobei die Unterseite des Fixierbandes die hydrophobe Beschichtung aufweist. Alternativ kann das Fixierband ein Schaumstoffband sein, welches auf seiner Unterseite die hydrophobe Beschichtung aufweist.

Wie oben erwähnt kann die hydrophobe Beschichtung die wenigstens eine Seite vollständig bedecken. Alternativ kann es von Vorteil sein, wenn die hydrophobe Beschichtung die wenigstens eine Seite nicht vollständig bedeckt. Durch die nicht von der Beschichtung bedeckten Bereiche kann dabei Feuchtigkeit von der Haut wegtransportiert werden bzw. verdunsten, was in einigen Anwendungen von Vorteil sein kann. Hierbei kann es von Vorteil sein, wenn zumindest die Randbereiche der beiden Längskanten der wenigstens einen Seite des Fixierbandes vollständig von der hydrophoben Beschichtung bedeckt sind. Es hat sich gezeigt, dass insbesondere durch die Längskanten der Fixierbänder ein erhöhtes Verletzungsrisiko für die Haut gegeben sein kann, beispielsweise durch Einschneiden der Kanten oder dergleichen. Dieses Problem kann vermieden werden, wenn die Beschichtung die Randbereiche der beiden Längskanten und damit auch die beiden Längskanten selber vollständig bedeckt. Hierbei werden zusätzliche Vorteile erzielt, wenn die hydrophobe Beschichtung im Bereich der Längskanten verdickt ist, da hierdurch der Schutz der Haut vor Verletzung weiter erhöht wird.

Wie oben erwähnt, kann es von Vorteil sein, wenn die hydrophobe Beschichtung die wenigstens eine Seite nicht vollständig bedeckt. Hierbei sind verschiedene Ausgestaltungen der Beschichtung denkbar, durch welche Teilbereiche der Seite nicht bedeckt sind. Eine vorteilhafte Ausgestaltung ist hierbei, wenn die hydrophobe Beschichtung durch zwei sich in Längsrichtung des Fixierbandes erstreckende parallele Streifen gebildet ist. Besonders vorteilhaft ist, wenn die beiden parallelen Streifen, wie oben erwähnt, die Randbereiche der beiden Längskanten vollständig bedecken und gegebenenfalls zusätzlich eine Verdickung im Bereich der Längskanten aufweisen. Damit wird das Fixierband rutschfest, ohne ein Verletzungsrisiko für die Haut eines Patienten darzustellen, und bietet gleichzeitig durch den freiliegenden Mittelbereich eine Möglichkeit zum Abtransport von Feuchtigkeit von der Haut. Vorteilhafterweise weisen die beiden Streifen jeweils eine konstante und gleiche Breite auf. Der zwischen den beiden Streifen frei liegende Mittelbereich ohne Beschichtung kann eine Breite aufweisen, die etwa der Breite der Streifen entspricht, kann aber auch eine Breite aufweisen, die größer oder kleiner als diejenige der beiden Streifen ist.

Besonders vorteilhaft ist es, wenn die hydrophobe Beschichtung eine silikonierte Beschichtung ist. Denn die damit bestehende Silikonauflage verbessert die unmittelbare Anhaftung des Fixierbandes am Endotrachealtubus infolge des erhöhten Reibkoeffizienten zwischen Beschichtung und dem Kunststoffmaterial des Endotrachealtubus. Der Einsatz eines zusätzlichen Klebers wird somit entbehrlich. Zusätzlich macht eine Beschichtung unter Verwendung von Silikon diese hypoallergen. Das Fixierband ermöglicht durch eine silikonisierte Beschichtung an der auf der Haut des Patienten anliegenden Seite auch eine bessere Haftung, also die Gefahr einer Lageveränderung des Fixierbandes selbst entlang des Halses ist damit erheblich verringert. Damit ist ein sicherer Sitz des Fixierbandes gewährleistet.

Daneben ist auch denkbar, anstelle der silikonisierten Beschichtung eine sonst gummierte Beschichtung zu verwenden zum Beispiel unter Einsatz von Polypropylen, jedoch mag aus gesundheitlichen Gründen eine Beschichtung aus Polyurethan ausscheiden.

Ein Fixierband, welches wenigstens teilweise aus hydrophobem Material gefertigt ist, bietet den Vorteil, keine Flüssigkeit auf zu nehmen. Günstigerweise ist es ein aus Nylonfäden gewebtes Band. Damit kann das Fixierband nicht aufquellen und hat keine Schwammwirkung. Das Fixierband muss so, obschon es in bestimmten Situationen mit Flüssigkeiten in Berührung kommt, nicht vorzeitig gewechselt werden, da es kein Wasser aufnimmt. Außerdem ist Nylon sehr zugstabil und dehnt sich nicht. So ist gewährleistet, dass der gesetzte Endotrachealtubus in der Lage stabil und sicher bleibt. Neben dem Einsatz von Nylon, was sich als besonders vorteilhaft herausgestellt hat, ist auch der Einsatz anderer hydrophober Materialien denkbar, sofern diese als Band eingesetzt werden können.

Um eine besonders gute Haltewirkung nach Anbringen des Fixierbandes am Endotrachealtubus bzw. am Kopf des Patienten zu erzielen, ist es vorteilhaft, das Fixierband mit einer Einschlitzung zu versehen, welche dreiecksförmig ist, wobei eine Seite des die Einschlitzung bildenden Dreiecks rechtwinkelig zu einer gedachten Längsachse entlang des Fixierbandes ausgerichtet ausgestaltet ist. Wird das Fixierband beim Anlegen durch die so gestaltete Einschlitzung an der rechtwinkeligen angeordneten Seite gefädelt und das Fixierband angezogen, reißt das Fixierband an den beiden äußeren Enden der genannten Seite ein. Die durch das Einfädeln entstandene Schlinge wird durch das Einreißen an der dreiecksförmigen Einschlitzung über die damit erzeugte Kerbwirkung festgezurrt, was ein ungewolltes Öffnen regelmäßig verhindert.

Wenn entlang des Fixierbandes wenigstens zwei Einschlitzungen in Abstand zueinander im Bereich einen freien Endes des Fixierbandes vorhanden sind, besteht der Vorteil, dass damit die Länge des Fixierbandes verschieden variiert werden kann, ähnlich einem Gürtel mit mehreren Gürtellöchern.

Vorteilhaft ist weiter, das Fixierband mittels einfach lösbaren Verbindungen, z. B. Klettverbindungen, an zwei sich am Patienten befindlichen Befestigungsplatten anzubringen. Damit wird der Patient insoweit geschont, als das Fixierband, welches den Endotrachealtubus hält, an den Befestigungsplatten am Patienten, vorzugsweise im Kopf- bzw. im Halsbereich selbst fixiert wird. Über die einfach lösbare Verbindung zu den Befestigungsplatten kann eine Lageveränderung des Fixierbandes und damit des Endotrachealtubus vorgenommen werden, ohne dass der Patient übermäßig strapaziert wird.

Eine vorteilhafte Ausgestaltungsform ist wie folgt beispielhaft dargestellt.

Es zeigen
Figur 1 eine Draufsicht auf das Fixierband,
Figur 2a zeigt eine Draufsicht auf einen Teil der Unterseite eines Ausführungsbeispiels des erfindungsgemäßen Fixierbandes,
Figur 2b zeigt einen Querschnitt durch das Ausführungsbeispiel von Figur 2a, und
Figur 3 eine Ansicht des angelegten Fixierbandes am Patienten.

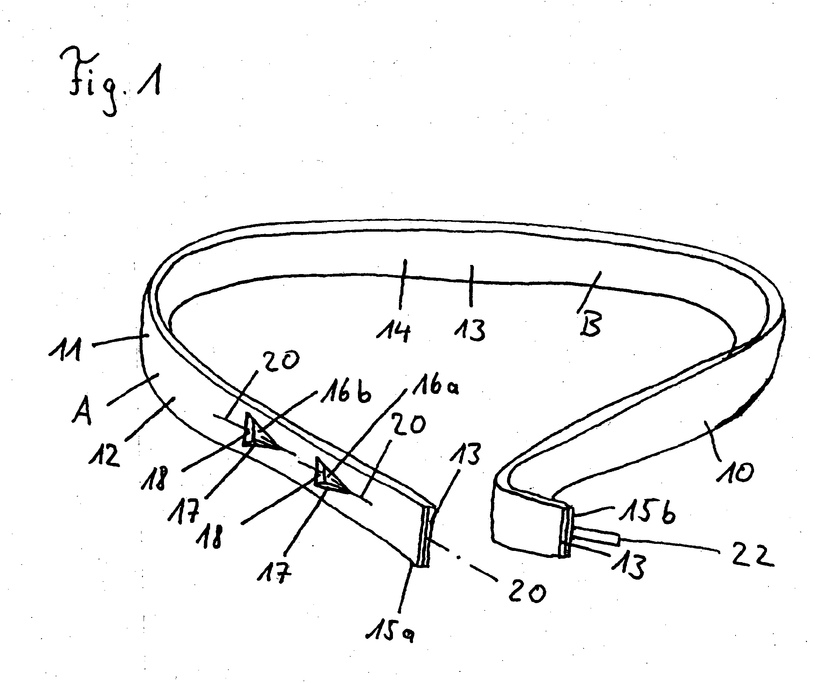

Figur 1 zeigt das Fixierband 10. Es besteht aus einem Band 11, welches zur Oberseite A hin mit einer Veloursschicht 12 ausgestaltet ist und zur Unterseite B hin eine hydrophobe Beschichtung 13 aufweist, welche hier im Fall als Silikonschicht 14 ausgestaltet ist. Anstelle der Veloursschicht 12 kann das Band eine Klettschicht aufweisen. Alternativ kann das Band 11 aus Schaumstoff oder Velours bestehen. Entlang einer gedachten Längsachse 20 befinden sich im Bereich eines freien Endes 15a des Fixierbandes 10 in Abstand zueinander zwei dreiecksförmige Einschlitzungen 16a, 16b. Dabei ist eine Seite 18 des jeweils die Einschlitzung 16a, 16b bildenden Dreiecks 17 rechtwinkelig zu einer gedachten Längsachse 20 entlang des Fixierbandes 10 ausgerichtet.

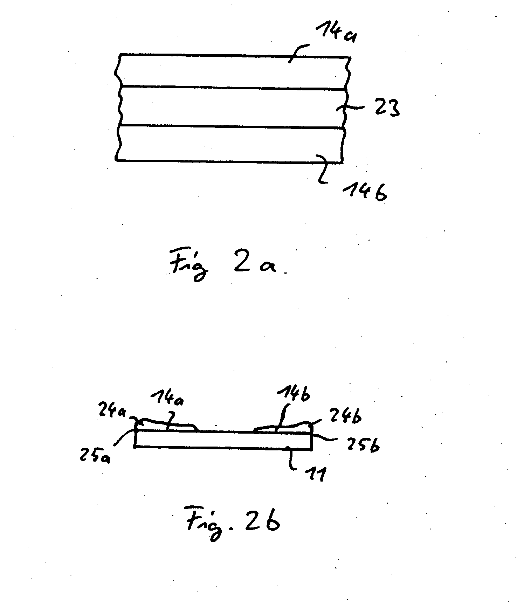

Figur 2a zeigt eine Draufsicht auf die Unterseite B eines Ausschnittes aus einer vorteilhaften Ausgestaltung des erfindungsgemäßen Fixierbandes 10. Die vorteilhafte Ausgestaltung des Fixierbandes 10 weist eine hydrophobe Beschichtung 13 auf, die die Unterseite B nicht vollständig bedeckt. Insbesondere besteht die Beschichtung aus zwei sich in Längsrichtung, d.h. in Richtung der Längsachse 20, erstreckende parallele Streifen 14a und 14b. Die beiden parallelen Streifen 14a und 14b sind derart an der Unterseite B angeordnet, dass sie die Randbereiche der beiden Längskanten 25a und 25b des Bandes 11 vollständig bedecken, wie es in Figur 2b zu erkennen ist, welche einen Querschnitt des in Figur 2a gezeigten Ausführungsbeispieles darstellt. Dabei sind die Längskanten 25a und 25b von den Streifen 14a und 14b überdeckt, oder zumindest fluchten sie mit den Seiten des Fixierbandes 10 dergestalt, dass die Längskanten 25a und 25b nicht mehr spürbar bzw. sichtbar sind und die Streifen 14a und 14b mit den Seiten des Fixierbandes 10 eine durchgehende Fläche bilden. Zwischen den parallelen Streifen 14a und 14b ist ein Mittelbereich 23 ohne Beschichtung freigelegt, durch welchen Feuchtigkeit von der Haut eines Patienten nach Außen abtransportiert werden kann. Die beiden Streifen 14a und 14b können jeweils eine konstante und gleiche Breite aufweisen. Es sind jedoch auch Ausgestaltungen denkbar, in denen die beiden Streifen 14a und 14b zwar jeweils eine konstante Breite, aber unterschiedliche Breiten aufweisen. Der Mittelbereich 23 kann eine Breite aufweisen, die etwa der Breite der beiden Längsstreifen 14a und 14b entspricht, d.h. für den Fall dass die beiden Streifen 14a und 14b eine etwa gleiche Breite aufweisen. Alternativ kann der Mittelbereich eine Breite aufweisen, die größer oder kleiner als die Breite der beiden Streifen 14a und 14b ist. Im Querschnitt der Figur 2b ist zu erkennen, dass die beiden Streifen 14a und 14b jeweils an den Längskanten 25a und 25b eine Verdickung 24a bzw. 24b aufweisen. Hierdurch wird ein potentielles Verletzungsrisiko für die Haut eines Patienten weiter verringert. Die Verdickungen 24a und 24b sind dabei vorteilhafterweise als abgerundete Erhebungen ausgebildet. Die Verdickungen 24a und 24b können dabei so ausgestaltet sein, dass ihre Außenseiten mit den Seiten des Fixierbandes 10 fluchten, oder dass sie über die Seiten des Fixierbandes 10 nach außen überstehen, um die Längskanten 25a und 25b abzudecken und Verletzungen von Patienten zu vermeiden.

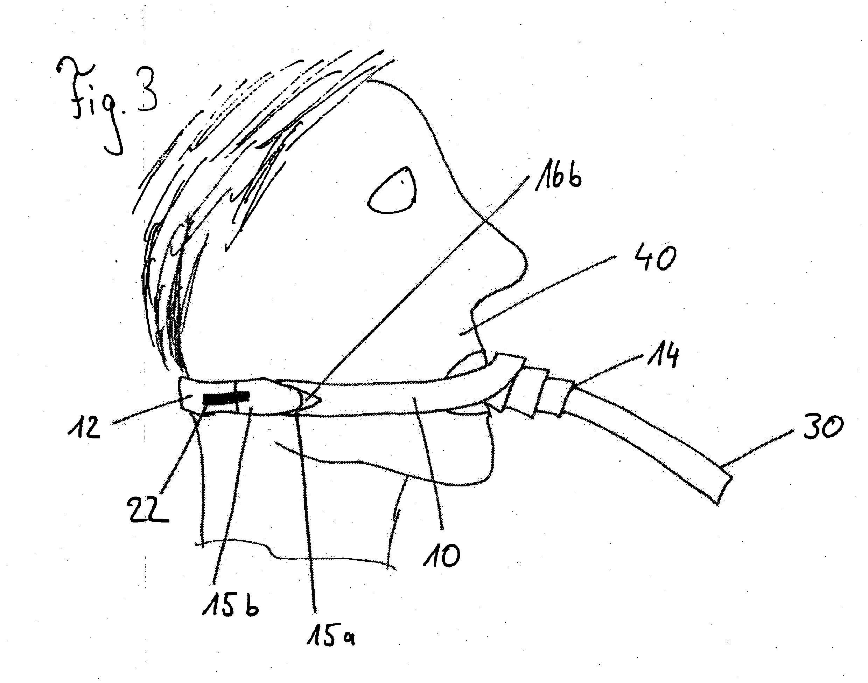

Wie in Figur 3 dargestellt, wird das Fixierband 10 mit der Silikonschicht 14 um einen Endotrachealtubus 30 unter Spannung geschlungen. Dabei haftet die Silikonschicht 14 an dem Endotrachealtubus 30 ohne einen weiteren Einsatz von Kleber. Dieser ist wegen der Haftreibung der Silikonschicht 14 am Kunststoff des Endotrachealtubus 30 möglich. Die vom Endotrachealtubus 30 weglaufenden freien Enden 15a, 15b des Fixierbandes 10 werden jeweils um den Kopf 40 des Patienten geschlungen, wobei das Fixierband 10 mit der Silikonschicht 14 unmittelbar auf der Haut des Patienten anliegt. Dann wird ein freies Ende 15b durch die dreiecksförmige Einschlitzung 16b des anderen freien Endes 15a gefädelt und in entgegengesetzter Richtung zurückgeführt. Dies erfolgt unter Spannung des Fixierbandes 10. Dadurch reißt das Fixierband 10 an der dreiecksförmigen Einschlitzung 16b und wird über die damit erzeugte Kerbwirkung festgezurrt. Dies verhindert ein ungewolltes Öffnen der dadurch gebildeten Schlinge des Fixierbandes 10 um den Kopf 40 des Patienten.

An dem freien Ende 15b befindet sich ein Klettstreifen 22, der in die Veloursschicht oder Klettschicht 12 greift. Somit ist das freie Ende 15b an dem Fixierband 10 befestigt. Eine sichere Fixierung des Endotrachealtubus 30 ist damit erreicht. Selbst bei schwitzenden Patienten ist keine ungewollte Lageveränderung des Fixierbandes 10 möglich, was durch die unmittelbar auf der Haut anliegende Silikonbeschichtung 14 verstärkt wird. Weiter kann durch die hydrophobe Silikonbeschichtung 14 in dem Bereich, in der sie aufliegt, keine Feuchtigkeit aus dem Fixierband 10 auf die Haut übertreten. Es ist dadurch auch besonders hautfreundlich. Da sich das Fixierband 10 auch nicht mit Flüssigkeiten vollsaugen kann, ist der Tragekomfort erheblich verbessert.

Das erfindungsgemäße Fixierband 10 kann auch ohne die Einschlitzungen 16a, 16b ausgestaltet sein. Die beiden Enden des Fixierbandes 10 werden in diesem Fall durch geeignete Befestigungsmittel miteinander verbunden, z. B. durch ein Klettplättchen oder dergleichen, welche mit der Veloursschicht oder Klettschicht 12 der Oberseite A der beiden Enden verbunden wird.

## Patentansprüche

1. Fixierband (10) für einen Endotrachealtubus, wobei das Fixierband (10) eine Oberseite (A) und eine unmittelbar auf die Haut des
Patienten aufzulegende Unterseite (B) aufweist, **dadurch gekennzeichnet, daß** nur auf der Unterseite (B) eine hydrophobe Beschichtung (13) vorgesehen ist.

2. Fixierband (10) nach Anspruch 1,
**dadurch gekennzeichnet, dass**
die Oberseite des Fixierbandes (10) als ein Teil eines Klettverschlusses ausgebildet ist und die Unterseite (B) die hydrophobe Beschichtung aufweist.

3. Fixierband (10) nach Anspruch 1,
**dadurch gekennzeichnet, dass**
das Fixierband ein Schaumstoffband ist, welches auf seiner Unterseite (B) die hydrophobe Beschichtung aufweist.

4. Fixierband (10) nach einem der Ansprüche 1 bis 3,
**dadurch gekennzeichnet, dass**
die hydrophobe Beschichtung die wenigstens eine Seite vollständig bedeckt.

5. Fixierband (10) nach einem der Ansprüche 1 bis 3,
**dadurch gekennzeichnet, dass**
die hydrophobe Beschichtung die wenigstens eine Seite nicht vollständig bedeckt.

6. Fixierband (10) nach Anspruch 5,
**dadurch gekennzeichnet, dass**
die Randbereiche der beiden Längskanten der wenigstes einen Seite vollständig von der hydrophoben Beschichtung bedeckt sind.

7. Fixierband (10) nach Anspruch 6,
**dadurch gekennzeichnet, dass**
die hydrophobe Beschichtung im Bereich der Längskanten verdickt ist.

8. Fixierband (10) nach Anspruch 5, 6 oder 7,
**dadurch gekennzeichnet, dass**
die hydrophobe Beschichtung durch zwei sich in Längsrichtung des Fixierbandes erstreckende parallele Streifen gebildet ist.

9. Fixierband (10) nach Anspruch 8,
**dadurch gekennzeichnet, dass**
die beiden Streifen jeweils eine konstante und gleiche Breite aufweisen und zwischen sich einen Mittelbereich ohne Beschichtung freilegen.

10. Fixierband (10) nach Anspruch 9,
**dadurch gekennzeichnet, dass**
der Mittelbereich eine Breite aufweist, die etwa der Breite von jedem der Streifen entspricht.

11. Fixierband (10) nach einem der Ansprüche 1 bis 10,
**dadurch gekennzeichnet, dass**
die hydrophobe Beschichtung (13) eine silikonierte Beschichtung (14) ist.

12. Fixierband (10) nach einem der Ansprüche 1 bis 11,
**dadurch gekennzeichnet, dass**
das Fixierband (10) wenigstens teilweise unter Verwendung von hydrophobem Material gefertigt ist.

13. Fixierband nach Anspruch 12,
**dadurch gekennzeichnet, dass**
das hydrophobe Material Nylon in Form von einem aus Nylonfäden gewebten Band ist.

14. Fixierband nach einem der Ansprüche 1 bis 13,
**dadurch gekennzeichnet, dass**
eine dreiecksförmige Einschlitzung (17) aufweist, wobei eine Seite (18) des die Einschlitzung bildenden Dreiecks (17) rechtwinkelig zu einer gedachten Längsachse (20) entlang des Fixierbandes (10) ausgerichtet ist.

15. Fixierband (10) nach einem der Ansprüche 1 bis 14,
**dadurch gekennzeichnet, dass**
entlang des Fixierbandes (10) wenigstens zwei Einschlitzungen (16a, 16b) in Abstand zueinander im Bereich einen freien Endes (15a) des Fixierbandes (10) vorhanden sind.

16. Fixierband (10) nach einem der Ansprüche 1 bis 15,
**dadurch gekennzeichnet, dass**
das Fixierband (10) mittels einfach lösbaren Verbindungen an zwei sich am Patienten befindlichen Befestigungsplatten angebracht werden kann.

## Claims

1. A fixing ribbon (10) for an endotracheal tube, wherein the fixing ribbon (10) provides an upper side (A) and a lower side (B) to be placed directly onto the patient's skin, **characterised in that**
a hydrophobic coating (13) is provided only on the lower side (B).

2. The fixing ribbon (10) according to claim 1,
**characterised in that**
the upper side of the fixing ribbon (10) is embodied as a hook and loop fastening and the lower side (B) provides the hydrophobic coating.

3. The fixing ribbon (10) according to claim 1,
**characterised in that**
the fixing ribbon is a foam-material ribbon which provides the hydrophobic coating on its lower side (B).

4. The fixing ribbon (10) according to any one of claims 1 to 3, **characterised in that**
the hydrophobic coating completely covers the at least one side.

5. The fixing ribbon (10) according to any one of claims 1 to 3, **characterised in that**
the hydrophobic coating does not completely cover the at least one side.

6. The fixing ribbon (10) according to claim 5,
**characterised in that**
the edge regions of the two longitudinal edges of the at least one side are completely covered by the hydrophobic coating.

7. The fixing ribbon (10) according to claim 6, **characterised in that**
the hydrophobic coating is thickened in the region of the longitudinal edges.

8. The fixing ribbon (10) according to claim 5, 6 or 7, **characterised in that**
the hydrophobic coating is formed by two parallel strips extending in the longitudinal direction of the fixing ribbon.

9. The fixing ribbon (10) according to claim 8, **characterised in that**
the two strips each provide a constant and identical width and leave exposed between them a middle region without coating.

10. The fixing ribbon (10) according to claim 9, **characterised in that**
the middle region provides a width which corresponds approximately to the width of each of the strips.

11. The fixing ribbon (10) according to any one of claims 1 to 10,
**characterised in that**
the hydrophobic coating (13) is a siliconised coating (14).

12. The fixing ribbon (10) according to any one of claims 1 to 11,
**characterised in that**
the fixing ribbon (10) is manufactured at least in part with the use of hydrophobic material.

13. The fixing ribbon according to claim 12,
**characterised in that**
the hydrophobic material is nylon in the form of a ribbon woven from nylon threads.

14. The fixing ribbon according to any one of claims 1 to 13, **characterised in that**
a triangular slit (17) is provided, wherein one side (18) of the triangle (17) forming the slit is orientated at right angles to an imagined longitudinal axis (20) along the fixing ribbon (10).

15. The fixing ribbon (10) according to any one of claims 1 to 14,
**characterised in that**,
along the fixing ribbon (10), at least two slits (16a, 16b) are provided at a distance from one another in the region of a free end (15a) of the fixing ribbon (10).

16. The fixing ribbon (10) according to any one of claims 1 to 15,
**characterised in that**
the fixing ribbon (10) can be attached by means of two readily releasable connections to two fastening plates disposed on the patient.

## Revendications

1. Bande de fixation (10) pour une sonde endotrachéale, ladite bande de fixation (10) comportant une face supérieure (A) et une face inférieure (B) à poser directement sur la peau du patient, **caractérisée en ce qu'**un revêtement hydrophobe (13) est prévu uniquement sur la face inférieure (B).

2. Bande de fixation (10) selon la revendication 1, **caractérisée en ce que** la face supérieure de la bande de fixation (10) est réalisée comme une partie d'une fermeture autoagrippante, et la face inférieure (B) est munie du revêtement hydrophobe.

3. Bande de fixation (10) selon la revendication 1, **caractérisée en ce que** la bande de fixation est une bande en mousse, dont la face inférieure (B) est munie du revêtement hydrophobe.

4. Bande de fixation (10) selon l'une quelconque des revendications 1 à 3, **caractérisée en ce que** le revêtement hydrophobe recouvre entièrement ladite au moins une face.

5. Bande de fixation (10) selon l'une quelconque des revendications 1 à 3, **caractérisée en ce que** le revêtement hydrophobe ne recouvre pas entièrement ladite au moins une face.

6. Bande de fixation (10) selon la revendication 5, **caractérisée en ce que** les zones de bordure des deux bords longitudinaux de ladite au moins une face sont entièrement recouvertes par le revêtement hydrophobe.

7. Bande de fixation (10) selon la revendication 6, **caractérisée en ce que** le revêtement hydrophobe est épaissi dans la zone des bords longitudinaux.

8. Bande de fixation (10) selon la revendication 5, 6 ou 7, **caractérisée en ce que** le revêtement hydrophobe est formé par deux rubans parallèles qui s'étendent dans la sens longitudinal de la bande de fixation.

9. Bande de fixation (10) selon la revendication 8, **caractérisée en ce que** les deux rubans ont chacun une largeur constante et identique et libèrent entre eux une zone centrale sans revêtement.

10. Bande de fixation (10) selon la revendication 9, **caractérisée en ce que** la zone centrale a une largeur qui correspond sensiblement à la largeur de chacun des rubans.

11. Bande de fixation (10) selon l'une quelconque des revendications 1 à 10, **caractérisée en ce que** le revêtement hydrophobe (13) est un revêtement (14) siliconé.

12. Bande de fixation (10) selon l'une quelconque des revendications 1 à 11, **caractérisée en ce que** la bande de fixation (10) est réalisée au moins en partie moyennant l'utilisation d'un matériau hydrophobe.

13. Bande de fixation selon la revendication 12, **caractérisée en ce que** le matériau hydrophobe est du nylon sous la forme d'une bande tissée dans des fils de nylon.

14. Bande de fixation selon l'une quelconque des revendications 1 à 13, **caractérisée en ce qu'**elle comporte une entaille (17) triangulaire, un côté (18) du triangle (17) formant l'entaille étant perpendiculaire à un axe longitudinal (20) imaginaire le long de la bande de fixation (10).

15. Bande de fixation (10) selon l'une quelconque des revendications 1 à 14, **caractérisée en ce que** le long de la bande de fixation (10) sont réalisées au moins deux entailles (16a, 16b) situées à distance l'une de l'autre dans la zone d'une extrémité libre (15a) de la bande de fixation (10).

16. Bande de fixation (10) selon l'une quelconque des revendications 1 à 15, **caractérisée en ce que** la bande de fixation (10) peut être attachée, au moyen d'assemblages aisément amovibles, sur deux plaques de fixation disposées sur le patient.
